(19) Europäisches Patentamt
European Patent Office
Office européen des brevets

(11) Veröffentlichungsnummer: **0 001 432**
**B1**

(12) **EUROPÄISCHE PATENTSCHRIFT**

(21) Anmeldenummer: **78101006.1**

(22) Anmeldetag: **27.09.78**

(51) Int. Cl.³: **C 07 C 53/02, C 07 C 51/09**

(54) **Verfahren zur Herstellung von Ameisensäure**

(30) Priorität: **01.10.77 DE 2744313**

(43) Veröffentlichungstag der Anmeldung:
**18.04.79 Patentblatt 79/08**

(45) Bekanntmachung des Hinweises auf die Patenterteilung:
**03.09.80 Patentblatt 80/18**

(84) Benannte Vertragsstaaten:
**BE DE FR GB NL**

(56) Entgegenhaltungen:
**DE - B - 2 407 157**
**GB - A - 839 371**
**GB - A - 1 460 491**
**US - A - 2 511 198**

(73) Patentinhaber: **BASF Aktiengesellschaft**
**Carl-Bosch-Strasse 38**
**D - 6700 Ludwigshafen (DE)**

(72) Erfinder: **Hohenschutz, Heinz, Dr.**
**Leibnizstrasse 12**
**D - 6800 Mannheim 1 (DE)**
**Schmidt, Johannes, E., Dr.**
**Pariser Strasse 23**
**D - 6700 Ludwigshafen (DE)**
**Kiefer, Hans, Dr.**
**Im Sandgarten 5**
**D - 6706 Wachenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 432 B1

Verfahren zur Herstellung von Ameisensäure

Die vorliegende Erfindung betrifft ein neues Verfahren zu Herstellung von Ameisensäure durch Hydrolyse von Methylformiat.

Aus "Ullmanns Enzyklopädie der technischen Chemie", 4. Auflage, Band 7, Seite 365 ist es bekannt, Ameisensäure durch Acidolyse von Formamid mit Schwefelsäure herzustellen. Dieses im großtechnischen Maßstab ausgeübte Verfahren hat jedoch den Nachteil, daß man hierbei stöchiometrische Mengen an Ammoniumsulfat als Zwangsanfall erhält.

Trotz dieses Nachteils hat die ebenfalls bekannte (Ullmann cit, S. 366), auf den ersten Blick wesentlich günstiger erscheinende Hydrolyse von Methylformiat

$$HCOOCH_3 + H_2O \rightleftharpoons HCOOH + CH_3OH$$

keinen Eingang in die Technik gefunden, und zwar hauptsächlich wegen der hohen Geschwindigkeit der Rückveresterung, welche durch die katalytisch wirkende starke Ameisensäure bedingt wird. Zwar kann man die Rückveresterung nach dem speziellen Destillationsverfahren der DE—PS 24 07 157 weitgehend unterdrücken, jedoch erfordert dieses Verfahren etwa 7 bis 8 t Dampf pro Tonne Ameisensäure, so daß es allein aus diesem Grunde wirtschaftlich kaum mehr in Betracht kommt. Außerdem erhält man nur das Ameisensäure/Wasser-Azeotrop, also lediglich eine rund 75 gew.%ige Säure, für die im Gegensatz zur reinen oder hochkonzentrierten Säure nur wenig Bedarf besteht.

Der Erfindung lag daher die Aufgabe zugrunde, die Ameisensäure auf wirtschaftlichere Weise zugänglich zu machen.

Es wurde gefunden, daß man Ameisensäure durch Hydrolyse von Methylformiat sehr vorteilhaft erhält, wenn man die Hydrolyse in Gegenwart von 0,5 bis 3,0 mol pro Mol Methylformiat einer Stickstoffbase mit ein oder mehreren basischen Stickstoffatomen welche keine wasserstoffatome tragen vornimmt, welche unter Normaldruck mindestens bei 180°C siedet und welche einen pKa-Wert von 4 bis 9 hat.

Es wurde weiterhin gefunden, daß sich für diesen Zweck Imidazolderivate der allgemeinen Formel I

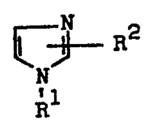

I,

in der $R^1$ für einen Kohlenwasserstoffrest mit 1 bis 12 C-Atomen steht und in der $R^2$ Wasserstoff oder einer der Reste $R^1$ ist, wobei die Anzahl der C-Atome von $R^1$ und $R^2$ zusammen 4 bis 12 beträgt, besonders gut eignen.

Als Kohlenwasserstoffreste in den Imidazolderivaten I kommen allgemein Alkylrest mit 1 bis 8 C-Atomen, der Cyclopentyl- und Cyclohexylrest sowie die Phenylgruppe und die Methylphenylgruppen in Betracht. Besonders bewählt haben sich hierunter solche Imidazolderviate, in dene $R^1$ ein $C_4$- bis $C_{10}$-n-1-Alkylrest und $R^2$ Wasserstoff oder eine Methylgruppe ist. Genannt seien beispielsweise 1-(n-1-Butyl)-imidazol (pKa 5,9), 1-(n-1-Pentyl)-imidazol (pKa 5,9), 1-(n-1-Decyl)-imidazol (pKa 5,75), 1-(n-1-Butyl)-2-methylimidazol (pKa 7,0) und 1-(n-1-Pentyl)-2-methylimidazol (pKa 6,85) und Chinolin (pKa 4,7).

Zur Definition der pKa-Werte, die ein Maß dür die Basenstärke sind, sei z.B. auf Landoldt-Börnstein, 6. Aufl., 7. Teil, II. Bd., Seite 900 ff. verwiesen. Die erfindungsgemäß zu verwendenden Basen sind entweder handelsüblich oder in bekannter Weise erhältlich. Da sie bei der Herstellung der Ameisensäure praktisch nicht verbraucht werden, fällt ihr Preis nicht ins Gewicht.

Die vorteilhafte Wirkung der Basen beruht darauf, daß sie mit der Ameisensäure eine salzartige Bindung eingehen. Diese Bindung ist einerseits stark genug, um die Ameisensäure aus dem Hydrolysegleichgewicht zu ziehen und andererseits ist sie schwach genug, daß sich die Ameisensäure ohne Zersetzung und ohne besonderen Energieaufwand wieder leicht von der Base abdestillieren läßt.

Das Molverhältnis von Methylformiat zu monofunktioneller Base — die Imidazolderivate I verhalten sich monofunktionell, das 1,2-Diimidazolyläthan beispeilsweise hingegen bifunktionell — beträgt vorzugsweise 1:1. zur Vervollständigung der Hydrolyse kann sich jedoch ein Basenüberschuß von insbesondere 0,5 bis 1 Mol empfehlen, wogegen höhere Überschüsse nur noch unwesentliche Vorteile bringen. Auch mit unterstöchiometrischen Mengen an der Base — in diesem Fall bleibt ein Teil der Ameisensäure ungebunden — läßt sich das Verfahren noch wirtschaftlich gestelten, besonders wenn man keine wasserfreie Säure herstellen will. Infolge der Gegenwart der Base reicht eine zur Menge des Methylformiats äquimolare Menge an Wasser zur Erzeilung befriedigender Umsätze aus. Größere Mengen an Wasser steigern den Umsatz des Methylformiats, jedoch ergibt sich ein erhöhter Dampfverbrauch bei der dann erforderlichen Aufkonzentrierung der Ameisensäurelösung.

Da das Methylformiat bei 32°C siedet, Methanol hingegen bei 65°C, ist es nicht möglich, das Methanol während der Reaktion laufend destillativ aus dem Hydrolysegleichgewicht zu entfernen. Man nimmt die Hydrolyse daher unter Vorgabe aller Komponenten bei einem Druck von 5 bis 15 bar sowie

den entsprechenden Temperaturen von 80 bis 150°C vor und führt sie zu einem Umsatz von etwa 60 bis 95%. Danach entspannt man das Reaktionsgemisch und gibt es zweckmäßigerweise hierbei unmittelbar in einen Kolonne, von der man das nicht umgesetzte Methylformiat sowie das Methanol als Kopfprodukte abzieht, die wie üblich in einer weiteren Kolonne in ihre Komponenten aufgetrennt werden. Die Sumpfprodukte der ersten Kolonne, Nämlich Wasser, die Base und das Addukt aus Ameisensäure und der Base, werden in einer Entwässerungskolonne bei 200 bis 400 Torr zunächst von Wasser befreit, bevor man aus dem Sumpfprodukt der Entwässerungskolonne in einer weiteren Kolonne die Ameisensäure bei 20 bis 100 Torr von der Base bzw. dem Addukt aus Ameisensäure und Base abzieht.

Sieht man von der erfindungsgemäßen Manßnahme, der Hydrolyse in Gegenwart der Base, ab, entsprechen alle übrigen Verfahrensbedingungen den bekannten Techniken oder könnén ohne weiteres von den bekannten Techniken abgeleitet werden. Dies gilt auch für diejenigen Maßnahmen, welche durch die Verwendung der Basen bedingt werden. Demgemäß stellt der oben geschilderte Ablauf nur eine unter verschiedenen möglichen Varianten dar. Beispielsweise läßt sich das Verfahren, etwa unter Zungrundelegung der genannten Reaktionsbedingungen, auch diskontinuierlich gestalten.

Die Vorteile der erfindungsgemäßen Verfahrens liegen darin, daß man keine Nebenprodukte erhält, die nicht wieder in den Verfahrenskreislauf zurückgeführt werden könnten und daß man bei dem relativ geringen Energiebedarf von 2 bis 3 t Dampf pro Tonne Ameisensäure unmittelbar reine oder hochkonzentrierte wäßrige Ameisensäure erhält.

## BEISPIEL 1

In einer Versuchsanlage wurde einem Rohrreaktor von 5 l Inhalt stündlich eine homogene Mischung aus 2121 g 97-gew.%igem Methylformiat (34,3 mol Methylformiat, 2 mol Methanol), 617 g Wasser (34,3 mol) und 4742 g (34,4 mol) 1-(1-n-Pentyl)-imidazol zugeführt. Die Temperatur im Reaktor betrug 130°C und der Druck rung 10 bar. Der stündliche Reaktoraustrag setzte sich aus 1001 g (21,75 mol) Ameisensäure, 753 g (1255 mol) Methylformiat, 760 g (23,75 mol) Methanol, 226 g (1255 mol) Wasser und 4742 g (34,4 mol) der Base zusammen.

Das Reaktionsgemisch wurde in die Mitte einer Füllkörperkolonne von 8 cm Durchmesser und 150 cm Höhe entspannt, wo bei einer Kopftemperatur von 40°C und einem Rücklaufverhältnis von 0,5 stündlich ein Gemisch aus 760 g Methanol und 753 g Methylformiat abgezogen wurde. Dieses Gemisch wurde in einer weiteren Kolonne wie üblich in 752 g Methanol und 746 g Methylformiat getrennt. Das Methylformiat wurde in den Hydrolysereaktor zurückgeführt und das Methanol in die hier nicht näher beschriebene Synthesestufe für das Methylformiat.

Das basenhaltige Sumpfprodukt der ersten Kolonne wurde in einer Füllkörperkolonne (8 cm Durchmesser, 100 cm Höhe, Rücklaufverhältnis 0,5) bei 300 Torr entwässert. Das Wasser (226 g) wurde ebenfalls in den Hydrolysereaktor zurückgeführt.

Zur Abtrennung der Ameisensäure wurde das Sumpfprodukt der Entwässerungskolonne in einer Füllkörperkolonne (10 cm Durchmesser, 250 cm Höhe, Rücklaufverhältnis 2, Kopftemperatur 35°C) bei 45 Torr destilliert. Hierbei wurden pro Stunde 991 g reine Ameisensäure gewonnen. Dies entspricht einer ausbeute von 99%, bezogen auf einen Methylformiatumsatz von 64%. Die Base, die nur noch Spuren Ameisensäure enthielt, wurde quantitativ zurückgewonnen und dem Hydrolysereaktor wieder zugeführt. Der Dampfverbrauch für die gesamte Versuchsanlage betrug 2,2 t pro Tonne reine Ameisensäure.

## BEISPIEL 2

Es wurden einige Methylformiat-Umsätze in Abhängigkeit von der Art des Base und den Konzentrationsverhältnissen ermittelt, in dem jeweils 1 Mol Methylformiat mit Wasser und der Base in einem Rührautoklaven bei 130°C und rung 10 bar 5 Stunden lang gerührt wurden.

Die Ergebnisse gehen aus der folgenden Tabelle hervor:

| Versuch | Base | Wasser mol | Base | Umsatz % |
|---|---|---|---|---|
| a | 1-(n-1-Butyl)-imidazol | 1 | 1 | 76 |
| b | ,, | 0,67 | 1 | 82+ |
| c | 1-(n-1-Pentyl)-imidazol | 1 | 1 | 66 |
| d | ,, | 2 | 1 | 85 |
| e | 1-(n-1-Butyl)-2-methyl-imidazol | 1 | 1 | 75 |
| f | 1-(n-1-Pentyl)-2-methyl-imidazol | 1 | 1 | 77 |
| g | 1-(n-1-Decyl)-imidazol | 1 | 1 | 64 |
| h | 1,2-Di-(1-imidazolyl)-äthan | 1 | 1++ | 64 |
| i | ohne Base, zum Vergleich | 1 | – | 31 |

+ bezogen auf Wasser

++ Moläquivalente

**Patentansprüche**

1. Verfahren zur Herstellung von ameisensäure durch Hydrolyse von Methylformiat, *dadurch gekennzeichnet,* daß man die Hydrolyse in Gegenwart von 0,5 bis 3,0 mol pro Mol Methylformiat einer Stickstoffbase mit ein oder mehreren basischen Stickstoffatomen, welche keine wasserstoffatome tragen, vornimmt, welche unter Normaldruck bei 180°C oder darüber siedet und welche einen pKa-Wert von 4 bis 9 hat.

2. Verfahren nach Anspruch 1; *dadurch gekennzeichnet,* daß man als Base ein Imidazolderivat der allgemeinen Formel I verwendet

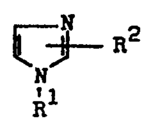 I,

in der $R^1$ für einen Kohlenwasserstoffrest mit 1 bis 12 C-Atomen steht und in der $R^2$ Wasserstoff oder einer der Reste $R^1$ ist, wobei die Anzahl der C-Atome von $R^1$ und $R^2$ zusammen 4 bis 12 beträgt.

**Revendications**

1. Procédé de préparation de l'acide formique par hydrolyse du formiate de méthyle, caractérisé en ce que l'hydrolyse est réalisée en présence, par mole de formiate de méthyle, de 0,5 à 3,0 moles d'une base azotée, comprenant un ou plusieurs atomes d'azote basiques ne portant pas d'atomes d'hydrogène, possédant un pKa compris entre 4 et 9 et un point d'ébullition égal ou superieur à 180°C à la pression normale.

2. Procédé suivant la revendication 1, caractérisé en ce que la base azotée est un dérivé de l'imidazole de la formule générale

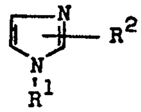 (I),

dans laquelle le symbole $R^1$ représente un groupe hydrocarboné en $C_1$ à $C_{12}$ et le symbole $R^2$ un atome d'hydrogène ou un groupe tel que défini pour $R^1$, la somme des atomes de carbone de $R^1$ et de $R^2$ étant comprise entre 4 et 12.

## Claims

1. A process for the manufacture of formic acid by hydrolyzing methyl formate, *characterized in that* the hydrolysis is carried out in the presence of form 0.5 to 3.0 moles, per mole of methyl formate, of a nitrogen base which contains one or more nitrogen atoms which do not bear hydrogen atoms, boils at not less than 180°C under atmospheric pressure and has pKa of from 4 to 9.

2. A process as claimed in claim 1, *characterized in that* the base used is an imidazole derivative of the general formula I

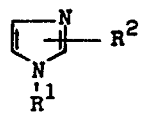

I,

where $R^1$ is a hydrocarbon radical of 1 to 12 carbon atoms and $R^2$ is hydrogen or one of the radicals $R^1$, the number of carbon atoms in $R^1$ and $R^2$ together being from 4 to 12.